Europäisches Patentamt

(19) European Patent Office

Office européen des brevets

(11) Publication number: **0 250 268**
**B1**

(12) # EUROPEAN PATENT SPECIFICATION

(45) Date of publication of the patent specification:
**05.12.90**

(51) Int. Cl.⁵: **C11D 17/00,** C11D 3/37,
A61L 9/01

(21) Application number: **87305486.0**

(22) Date of filing: **19.06.87**

(54) Toilet bowl cleaner.

(30) Priority: **20.06.86 US 876923**

(43) Date of publication of application:
**23.12.87 Bulletin 87/52**

(45) Publication of the grant of the patent:
**05.12.90 Bulletin 90/49**

(84) Designated Contracting States:
**AT BE CH DE ES FR GR IT LI LU NL SE**

(56) References cited:
**EP-A- 0 053 055**
**CH-A- 486 885**
**JP-A-58 025 398**
**JP-A-58 168 699**
**JP-A-59 024 797**
**JP-A-61 083 300**
**LU-A- 78 055**
**US-A- 4 043 931**

(73) Proprietor: **KIWI BRANDS INC, Rt. 662 North,
Douglassville Pennsylvania 19518(US)**

(72) Inventor: **Bunczk, Charles J., 3211 Sunset Avenue,
Norristown Pennsylvania 19401(US)**
Inventor: **Burke, Peter A., Two Surrey Lane,
Downingtown Pennsylvania 19335(US)**
Inventor: **Strauch, Edward R., 6105 Glen Road, Reading
Pennsylvania 19606(US)**

(74) Representative: **Burford, Anthony Frederick et al, W.H.
Beck, Greener & Co. 7 Stone Buildings Lincoln's Inn,
London WC2A 3SZ(GB)**

ACTORUM AG

## Description

The present invention relates to cake compositions which are useful for the treatment of the flush water of toilets. More particularly, the invention is concerned with a long lasting toilet tank dispenser which may be formed by casting and is responsive to the flushing of the toilet.

In treating toilet flush water with chemicals in order to produce desirable effects such as bowl aesthetics, cleaning, disinfection, deodorization, aerosol reduction, etc., it is desirable that the chemicals be dispensed into the flush water automatically each time the toilet is flushed. The prior art discloses numerous devices which have been designed for this purpose.

Particularly desirable devices are those comprising a solid cake composition. In this type of device, a measured amount of water enters the device during one flush cycle and remains in contact with the cake between flushes, thereby forming a concentrated solution of the composition which is dispensed into the flush water during the next flush. The advantages of such devices are that the chemical composition can be packaged and shipped in more concentrated form than aqueous solutions of the chemicals. Also, the problems of liquid spillage resulting from breakage of the dispensers during shipment or handling is eliminated.

Prior art surfactant cake compositions are disclosed in U.S.-A 4 308 625 (Kitko) and U.S.-A 4 043 931 (Jeffrey et al). These patents disclose a lavatory cleansing tablet which two or more non-ionic surfactants which includes the use of polyaloxylated alcohols.

U.S.-A 4 477 363 (Wong et al) discloses a solid cake comprising metal alkyl sulfate surfactant.

JP-A 58-168 699 (Japan Synthetic Rubber Co. et al) discloses a cast lavatory cleansing block containing 25 to 90 weight % of a mixture of polyethylene glycol or monoester thereof and polyethylene glycol diester. The preferred monoester and diester are the stearates and the exemplified stearates are polyethylene glycol 4000 and 6000 monostearates and polyethylene glycol 6000 distearate. The block may also contain inter alia a deodorising agent, detergent, colorant, filler, binder, aromatic, and dissolution rate adjuster.

JP-A 58-25 398 (Lion) discloses a cast lavatory cleansing block containing 40 to 98 weight % of polyethylene glycol diester, preferably distearate. Especially preferred diesters are polyethylene glycol 1500, 3000 and 9000 distearates. The block may also contain inter alia aromatic, colorant, surface-active agent, germicide, and builder.

JP-A-59-24 797 (Earth I) discloses a cast lavatory cleansing block containing at least 40% of a polyethylene glycol distearate having 91 to 230 moles ethylene oxide adduct groups and 0.1 to 60% of either polyoxyethylene sorbitan monostearate, 6 ethylene oxide or polyethylene sorbitan tristearate, 20 ethylene oxide. Exemplified polyethylene glycol distearates have 91, 160, 230 and 245 moles ethylene oxide. The block may also contain inter alia dye and fragrance.

JP-A 61-83 300 (Earth II) discloses a cast lavatory cleansing block containing 1–8 volume% 12-hydroxy stearic acid and/or 0.1–5% benzylidene sorbitol; 20–70% perfume; 10–30% coloring matter, and 15–60% surface active agent. The surface active agent can comprise a polyethylene glycol distearate. The block may also contain inter alia deodorant, bactericide and chlorine-remover.

U.S.-A 4 269 723 (Barford et al I) discloses a compressed lavatory cleansing block containing one or more organic surface active agents and one or more binders which binder(s) act as dissolution retarding agents and are selected from clays and water-soluble and water-dispersible gel-forming organic polymeric materials. Specified binders include alginates and carragheenates and specified surface active agents include alkylene oxide condensates of fatty acids.

G.B.-A-206I996A (Jeyes) is substantially the same as Barford et al I except that the block is formed by melting lower-melting point components, dispersing higher melting point and/or liquid components in the melt, and then casting into a mould.

U.S.-A-4460490 (Barford et al II) discloses a bi-component lavatory cleansing block comprising a shaped body formed of a slow-dissolving cleansing composition containing at least one surface active agent and a tablet comprising a bleaching agent embedded in or adhered to the shaped body. The shaped body preferably comprises one or more readily-soluble surface active agents in admixture with one or more solubility control agents. Specified solubility control agents include low ethoxylates of fatty acids and gel-forming gums, such as xanthan gum, or materials, such as alginates or carragheenates.

U.S.-A-4,3I0,434 (Choy et al) and U.S.-A-4,278,57I, (Choy), disclose surfactant cake compositions containing dyes and perfumes which can be utilized in the present invention. The surfactants provide cleaning and sudsing in the toilet bowl and also serve to dispense other components of the compositions such as dyes, perfumes, organic resins, etc.

Water-soluble inert salts such as alkali metal chlorides and sulfates are used in such compositions to act as a "filler" so that the composition can be formed into cakes of desirable size without using excessive amounts of active ingredients. The predominant ingredients of the cake compositions are usually the surfactant, perfume and the filler salt.

A major problem in this art has been short and/or erratic longevity of surfactant cakes because of rapid and uneven dissolution resulting in decreased cake stability and longevity.

It has been found that a cast solid cake composition which has a long and uniform block life can be provided where the composition comprises a polyethylene glycol distearate if said distearate has a specific water solubility and molecular weight range.

It is an object of the present invention to provide a solid cake which may be formed by casting and comprises a specific kind of polyethylene glycol distearate, which compositions are suitable for use for automatically dispensing cleansing agents into the toilet.

It is a further object of the present invention to provide a cast solid cake composition having relatively high melt temperatures and less block surface tackiness for improved processing.

It is a still further object of the present invention to provide a lavatory block which has a long and uniform block life that eliminates sluggish toilets.

It is a yet still further object of the present invention to provide a lavatory block which resists mounding and major fragmentation.

Other objects, advantages and novel features of the present invention will be apparent to those skilled in the art from the following description and appended claims.

The objectives of the invention are achieved by providing a solid unsupported cake composition which comprises polyethylene glycol distearate having a drop dissolution time of at least 5.5 hours according to the Distearate Dissolution Test (see Example 1 hereinafter) and a molecular weight from 3,000 to 12,000, preferably 7,000 to 9,000 and, as a binder, guar gum and, as a filler, sodium chloride, and optional ingredients selected from the groups consisting of fragrances dyes, binders, filler materials and mixtures thereof. Advantageously, the cake composition comprises from 8% to 35%, preferably 12% to 20%, by weight of said polyethylene glycol distearate.

It is known that polyethylene glycol distearate is extremely hydrophilic. Because of the extreme hydrophilic nature of the compound, it would be expected that the material would be very water soluble without any prolonged transition from solid to a liquid. It has been surprisingly found that the particular kind of polyethylene glycol distearate of the invention goes through a hydration stage forming a tenacious gel so as to provide the aforementioned extended block life relative to other formulations containing polyethylene glycol distearate of varying molecular weights.

It is critical in the present invention that the polyethylene glycol distearate which is utilized in the formulation of the cake composition has a drop dissolution time of at least 5.5 hours according to the Distearate Dissolution Test. It has been found that not all polyethylene glycol distearates having a molecular weight of about 3,000 to 12,000 possess such a characteristic. Their method of preparation appears to influence their solubility. Preferably, their preparation is according to the method of condensing a fatty acid with an alcohol as described by W.B. Satkowski et al in "Polyoxyethylene esters of Fatty Acids", Non-ionic Surfactants, M.J. Schick Ed. (Dekker, NY 1967) p. 142–174. For example, stearic acid having a molecular weight of 284.5 is reacted with a polyethylene glycol having a molecular weight range between 5500–8500 to form a polyethylene glycol distearate having a molecular weight range of about 6033–9033. Such compound having a dissolution time of at least 5.5 hours is suitable for use in the invention.

In order to improve the cake characteristics, it has been found advantageous to utilize in the cake composition a greater portion of polyethylene glycol distearate which has a molecular weight between 7,000 to 12,000. A lesser ortion of the polyethylene glycol distearate having a molecular weight between 3,000 to 7,000 preferably 3,000 to 4,000 in combination with the higher molecular weight polyethylene glycol distearate acids in preventing mounding and further acts as a binder.

The use of guar gum as the binder has been found to be most effective in retarding block dissolution and to reduce the problem of sluggish toilet behaviour. Usually, the guar gum is present in an amount of 3 to 35%, preferably 5 to 15%, by weight of the composition.

It has been found to be particularly advantageous to utilize guar gum together with sodium chloride as a filler since there is a synergistic viscosity increase of water that is not found with other fillers such as calcium sulfate. Additionally, there is an increase of the relative insolubility properties of the matrix in water.

As a further binding agent, the use of solid emollients have been found to be helpful to prevent the cake of the invention from mounding out. Suitable emollients include glyceryl monostearate, glyceryl monopalmitate, ethylene glycol stearate, propylene glycol monostearate, and the like, most preferably is glyceryl monostearate which provides a matrix to prevent mounding. The emollients may be utilized in amounts of up to 20% by weight, preferably 5% to 12%.

It has been found to be advantageous to utilize certain non-ionic surfactants in the cake formulation. Non-ionic surfactants that may be included are the condensation products of a long chain ethylene oxide moiety with an aliphatic alcohol preferably a primary or secondary aliphatic alcohol or an alkyl phenol. Preferably the primary and secondary alcohol contains 8 to 20 carbon atoms and the alkyl phenol-based moiety is one wherein the alkyl chain is straight or branched and contains 6 to 12 carbon atoms, preferably 6 to 9 carbon atoms.

Illustrative non-ionic surfactants having the desired characteristics for formulation are available on the market under the Registered Trade Marks "Neodol" (Shell Oil Company), "Tergitol" (by Union Carbide Company), and "Alfol" (Continental Oil Company). Specific examples include "Neodol 25–7" (linear $C_{12}$–$C_{15}$ primary alcohol condensed with 7 moles of ethylene oxide per mole of alcohol); "Neodol 54–7" (linear $C_{14}$–$C_{15}$ primary alcohol condensed with 7 moles of ethylene oxide per mole of alcohol); "Tergitol 15–S–7" (random secondary $C_{11}$–$C_{15}$ alcohol condensed with 7 moles of ethylene oxide per mole of alcohol); and "Alfol 1416–6.5" (primary $C_{14}$–$C_{16}$ alcohol condensed with 6.5 moles of ethylene oxide per mole of alcohol).

Such non-ionic surfactants act as coupling agents to provide an integration of the cake components and may be used in the amount of up to 40%, preferably 20 to 30%, by weight of the cake formulation.

Also useful to enhance the life of the cake are ethoxylated nonylphenols. The high ethoxylated nonylphenols, that is, those having over 20 moles of ethylene oxide per mole of phenol, provide slow dissolution of the cake formulation. Up to 10%, preferably up to 5%, by weight of ethoxylated nonylphenols is preferably utilized together with the ethoxylated aliphatic alcohols.

Sodium chloride is used in present compositions as a "filler" so that the composition can be formed into cakes of desired size without using excessive amounts of active ingredients. It is used alone or in combination with further salts in amounts up to 64% by weight.

Further inert salts (filler salts) used in the compositions of the present invention can be any water-soluble inorganic or organic salt or mixtures of such salts. For purposes of the present invention, "water-soluble" means having a solubility in water of at least 0.2 grams per hundred grams of water at 20°C. Examples of suitable salts include various alkali metal and/or alkaline earth metal sulfates, chlorides, borates, bromides, citrates, acetates, lactates, etc.

Specific examples of suitable further salts include calcium sulfate, potassium sulfate, sodium carbonate, lithium chloride, tripotassium phosphate, sodium borate, potassium bromide, potassium fluoride, sodium bicarbonate, calcium chloride, magnesium chloride, sodium citrate, sodium acetate, calcium lactate, magnesium sulfate and sodium fluoride. The preferred salts are the inorganic salts, especially the alkali metal sulfate and chlorides. The preferred further salt, because of its low cost, is calcium sulfate. The salts are present in the compositions herein at levels of from 20% to 64% by weight, preferably form 20% to 35%. Sodium chloride is utilized together with guar gum either alone or with other salts since the combination not only provides asynergistic viscosity increase of water and decreases the relative solubility properties of the matrix in water but also aids to prevent mounding.

Calcium sulfate is advantageously utilized together with sodium chloride because it has a low solubility level which is constant over the water temperature range likely to exist within toilet tanks.

Various optional materials may be included in the compositions herein.

Dyes may be included at levels of up to 15%, preferably 2.5% to 10% by weight. Examples of suitable dyes are Alizarine Light Blue B (C.I. 63 010), Carta Blue VP (C.I. 24 401), Acid Green 2G (C.I. 42 085), Astragon Green D (C.I. 42 040), Supranol Cyanine 7B (C.I. 42 675), Maxilon Blue 3RL (C.I. Basic Blue 80), Drimarine Blue Z-RL (C.I. Reactive Blue 18), Alizarine Light blue H-RL (C.I. Acid Blue 182), FD & C Blue No. 1, FD & C Green No. 3 and Acid Blue No. 9 (C.I. 42 090). Others are disclosed in the aforementioned Patent Nos. 4 310 434 and 4 477 363.

The cakes of the invention may also contain up to 15% by weight of a cationic quaternary ammonium salt.

It is known that the cationic quaternary ammonium salts which include a greater number of short-chain alkyl groups in the structure, incline toward better bacteriostatic properties. Specific examples of bacteriostatic agents that may be used in the compositions of this invention include di-isobutyl cresoxy ethoxy ethyl dimethyl benzyl ammonium chloride, di-isobutyl phenoxy ethoxy ethyl dimethyl benzyl ammonium chloride, myristyl dimethylbenzene ammonium chloride, benzalkonium chloride, cetyl pyridinium chloride, coconut dimethyl benzyl ammonium chloride, stearyl dimethyl benzyl ammonium chloride, alkyl dimethyl benzyl ammonium chloride, alkyl diethyl benzyl ammonium chloride, alkyl dimethyl benzyl ammonium bromide, di-isobutyl phenoxy ethoxy ethyl trimethyl ammonium chloride, di-isobutyl phenoxy ethoxy ethyl dimethyl alkyl ammonium chloride, methyl-dodecylbenzyl trimethyl ammonium chloride, cetyl trimethyl ammonium bromide, octadecyl dimethyl ethyl ammonium bromide, cetyl dimethyl ethyl ammonium bromide, octadecenyl-9-dimethyl ethyl ammonium bromide, dioctyl dimethyl ammonium chloride, dodecyl trimethyl ammonium chloride, octadecyl trimethyl ammonium chloride, octadecyl trimethyl ammonium bromide, hexadecynyl trimethyl ammonium iodide, octyltrimethyl ammonium fluoride, and mixtures thereof. Other water dispersible salts, such as the acetates, sulfates, nitrates, and phosphates, are effective in place of the halides, but the chlorides and bromides are preferred.

The cakes may also contain perfumes to impart an acceptable odor to the flushing water. The perfume may be in solid form and is suitably present in an amount up to l5%, preferably up to l0%, by weight. In this connection, it may be noted that the term "perfume" is intended to refer to any material giving an acceptable odor and thus materials giving a "disinfectant" odor such as essential oils, pine extracts, terpinolenes, ortho phenyl phenol or para-dichlorobenzene may be employed. The essential oils and pine extracts also contribute as plasticizers and are functional to a degree in extending block life.

Certain perfume materials may be added which additionally function to control the solubility of anionic sulfate surfactants. Examples of such perfume materials are isobornyl acetate, myrtenyl acetate and fenchyl acetate. Other suitable perfume or fragrances are disclosed in U.S. Patent No. 4 396 522 of Callicott et al.

The cake formulation may also contain other binding and/or plasticizing ingredients serving to assist in the manufacture thereof, for example, polypropylene glycol having a molecular weight from 3,000 to 10,000 in an amount up to 20% by weight, preferably 4% to 15% by weight of the mixture may be used. The polypropylene glycol reduces the melt viscosity, acts as a demolding agent and also acts to plasticize the block when the composition is prepared by a casting process. Other suitable plasticizers such as pine oil fractions, di-limonene, dipentene and the ethylene oxide-propylene oxide block copolymers may be utilized.

The blocks of the present invention can be produced by a variety of processes, e.g. casting/moulding process, by tablet compression process or by an extrusion process. The casting process being the preferred process of the invention.

The casting process which is well within the skill of those in the art involves the melting of the ingredients and then casting the melt into appropriate shaped moulds and allowing the melt to cool and solidify. The shaped tablets or blocks each suitably have a weight of from 20 to 150 grams, preferably from 30 to 70 grams.

The shaped tablets or blocks of the preferred embodiments of the invention comprise:
from 8% to 35% by weight of polyethylene glycol distearate having a molecular weight of 3,000–12,000 and a dissolution time of at least 5.5 hours according to the Distearate Dissolution Test; up to 35% by weight guar gum, up to 32% by weight sodium chloride; and at least one of:
up to 10% by weight of glyceryl monostearate;
up to 30% by weight of an ethoxylated $C_8$–$C_{20}$ aliphatic alchohol;
up to 10% by weight of ethoxylated nonylphenol;
from 4% to 15% by weight of plasticizer;
up to 32% by weight of calcium sulfate, and
up to 15% of disinfecting agents, coloring agents and/or fragrances.

In order that the invention may be better understood the following Examples are given by way of illustration only. In the Examples, all parts and percentages are by weight unless otherwise stated.

The following Examples are for compositions suited for forming shaped bodies of blocks by a casting/moulding operation.

EXAMPLE I

Distearate Dissolution Test

To determine the dissolution rate of polyethylene glycol distearate in water the test is preformed as follows:

A sample of the polyethylene glycol distearate is placed into a beaker and heated so as to form a melt. Using a 7.5 ml capacity polyethylene transfer pipet, one drop of the melt is placed in the centre of a petri dish. The drop is allowed to fully solidify for the minutes, then 75 ml of deionized water is added to the dish. The dish is monitored to determine the time required for the drop of surfactant to totally dissolve.

A dissolution time of at least 5.5 hours indicates that the polyethylene glycol distearate with the desired molecular weight is suitable for use in formulating the composition of the invention.

EXAMPLE II

A. Procedure for the Selection of Polyethylene Glycol 6000 Distearate (PEG 6000DS)

To determine the dissolution rate of polyethylene glycol distearate in water the test is performed as follows:

I. An aliquot of PEG 6000DS is placed in a beaker and melted.

2. Using a 7.5 ml polyethylene transfer pipet, a drop of the melt is transferred to a microscope glass slide, the weight of PEG 6000DS added being 0.02 + or -0.00I g using an analytical balance. The melt is allowed to solidify for ten minutes.

3. The glass slide is carefully placed in a I000 ml glass beaker containing 800 cc deionized water which is immersed in a 38°C water bath.

4. The beaker is monitored to determine the time necessary for the drop of PEG 6000DS to totally dissolve. A dissolution time of at least 5.5 hours indicated that the polyethylene glycol distearate was suitable for use in formulating the composition of the invention.

B. Preparation of Cake Composition Polyethylene glycol 6000 distearate from Part A2I%
Ethoxylated $C_{12}$-$C_{15}$ Linear, Primary Alcohol with 7EO29.5%
Ethoxylated Nonylphenol with I00 EO5.5%
Ethylene oxide-propylene oxide block copolymer (8500 MW 80% EO)3.5%
Acid Blue No.9 dye5.5%
Dipentene8.0%
Ortho-phenyl phenolI.0%
Guar gum5.5%
Sodium chloride20.5%

Into a first mixture vessel four-fifths of the ethoxylated $C_{12}$-$C_{15}$ linear primary alcohol is added and slowly heated with stirring. The polyethylene glycol 6000 distearate, ethoxylated nonylphenol and ethylene oxide-propylene oxide block copolymer are added and the mixture is heated with stirring to 7I°C to form a clear melt.

In a separate vessel, to the remaining one-fifth of the ethoxylated linear primary alcohol there is added with stirring the dye, the dipentene and the ortho-phenyl phenol. The mixture is then added to the first mixing vessel followed by the guar gum and the sodium chloride. The mixture is cooled to 57°C and poured into molds. After cooling to 5°C, the blocks are removed from the mold.

EXAMPLE III

Following the procedure of Example II, a shaped lavatory cake composition is prepared with the following ingredients:
Polyethylene glycol 6000 distearate9.0%
Glyceryl monostearate5.5%
Ethoxylated $C_{12}$-$C_{15}$ Linear, Primary Alcohol with 7EO20.0%
Ethoxylated Ceto Stearyl Alcohol with 50 EOI7.5%
Acid Blue No.9 dye5.5%
Terpinolene8.0%
Ortho-phenyl phenolI.0%
Guar gum9.5%
Sodium chloride24.0%

EXAMPLE IV

Following the procedure of Example II, a shaped cake composition is prepared with the following ingredients:
Polyethylene glycol 6000 distearate20.0%
Glyceryl monostearate - acid stableI0.0%
Ethoxylated aliphatic alcohol (Neodol 45-7)20.0%
Guar gum9.0%

Sodium chloride26.0%
Polypropylene glycol (PPGDI000)5.0%
Cetyl trimethyl ammonium bromidel.0%
Acid Blue No. 9 dye4.0%
Terpinolene5.0%

The composition had a melt viscosity of 2000 cps (2 Pa.s) at 49°C and a set point at 46°C. The shaped tablet had an in-tank life of more than 30 days and showed only slight mounding.

EXAMPLE V

Following the procedure of Example II, a shaped cake composition is prepared with the following ingredients:
Polyethylene glycol 6000 distearate20.0%
Glyceryl monostearate - acid stablel0.0%
Ethoxylated aliphatic alcohol (Neodol 45-7)2l.0%
Guar gum6.0%
Sodium chloride26.0%
Polypropylene glycol (PPGDI000)6.0%
Cetyl trimethyl ammonium bromidel.0%
Acid Blue No. 9 dye4.0%
Terpinolene6.0%

The composition had a melt viscosity of ll20 cps (l.2 Pa.s) at 5l°C and a set point at 46°C. The shaped tablet had an in-tank life of 20-40 days in 6 different toilets and mounded out after 30 days.

EXAMPLE VI

Following the procedure of Example II, a shaped cake composition is prepared with the following ingredients:
Polyethylene glycol 6000 distearatel6.5%
Glyceryl monostearate - acid stable5.5%
Ethoxylated aliphatic alcohol (Neodol 45-7)25.0%
Guar gum6.0%
Sodium chloride   32.0%
Polypropylene glycol (PPGD1000)   5.0%
Cetyl trimethyl ammonium bromide   1.0%
Acid Blue No. 9 dye   4.0%
Terpinolene   5.0%

The shaped composition had an in-tank life of about 25–30 days in 6 different toilets and showed only slight mounding.

EXAMPLE VIII

PROCEDURE FOR PRELIMINARY EVALUATION OF THE MOUNDING AND COUPLING PROPERTIES OF A TOILET BLOCK

To determine the potential for a block to (1) mound or spread out from its original shape; and (2) remain an integrated unit once the block has been immersed in water, a test is conducted as follows:

1. Two 2000 ml glass beakers are filled with tap water. One is placed in a refrigerator at about 5°C for at least four hours while the other beaker remains at ambient temperature.

2. One block from the sample lot is placed in each beaker. The blocks remain immersed in water overnight or about sixteen hours.

3. The blocks are then observed after the immersion period. The ambient temperature sample provides an indication of the degree of spreading or enlarging of the block base that may occur during immersion within a toilet tank. The 5°C sample provides an indication of the integration of the block components or tendency to dissolve in unison.

The principals, preferred embodiments and modes of operation of the present invention have been described in the foregoing specification. The invention which is intended to be protected herein, however, is not to be constructed as limited to particular forms disclosed, since these are to be regarded as illustrative rather then restrictive. Variations and changes may be made by those skilled in the art without departing from the scope of the invention as defined in the following claims.

**Claims**

1. A solid cake lavatory cleansing block composition comprising polyethylene glycol distearate having a molecular weight from 3,000 to 12,000, a binder, and a filler characterized in that the distearate has a drop dissolution time (time for a fully solidified melt phase drop dispensed from a 7.5ml pipette to completely dissolve in 75ml deionized water at ambient temperature) of at least 5.5 hours, the binder is guar gum and the filler is sodium chloride.

2. A cleansing block composition as claimed in Claim 1, wherein said polyethylene glycol distearate is present in an amount of 8 tc 35% by weight of the composition.

3. A cleansing block composition as claimed in Claim 2, wherein said polyethylene glycol distearate is present in an amount of 12 to 29% by weight.

4. A cleansing block composition as claimed in any one of the preceding Claim 3, wherein guar gum is present in an amount of 3 to 35% by weight of the composition.

5. A cleansing block composition as claimed in Claim 4, wherein the guar gum is present in an amount of 5 to 15% by weight.

6. A cleansing block composition as claimed in any one of the preceding claims, wherein sodium chloride is present in an amount of 20 to 64% by weight of the composition.

7. A cleansing block composition as claimed in Claim 6, wherein the sodium chloride is present in an amount of 20 to 35% by weight.

8. A cleansing block composition as claimed in any one of the preceding claims, wherein said polyethylene glycol distearate comprises a mixture of a major proportion of polyethylene glycol distearate having a molecular weight between 7,000 and 12,000 and a minor proportion of polyethylene glycol distearate having a molecular weight between 3,000 and 7,000.

9. A cleansing block composition as claimed in any one of the preceding claims further comprising a solid emollient in an amount of up to 20% by weight of the composition.

10. A cleansing block composition as claimed in Claim 9, wherein the solid emollient is selected from glyceryl monostearate, glyceryl monopalmitate, ethylene glycol stearate, and propylene glycol monostearate.

11. A cleansing block composition as claimed in any one of the preceding claims, including a surfactant selected from ethoxylated aliphatic (C$_8$-C$_{20}$) alcohols and ethoxylated C$_6$-C$_{12}$ alkyl phenols.

12. A cleansing block composition as claimed in any one of the preceding claims, including a mixture of ethoxylated nonylphenols having more than 20 moles of ethylene oxide per mole of phenol.

13. A cleansing block composition as claimed in any one of the preceding claims, including a cationic quaternary ammonium salt.

14. A cleansing block composition as claimed in any one of the preceding claims, including a plasticizer selected from polypropylene glycol, dipentene, pine oil fractions, d-limonene and ethylene oxide-propylene oxide copolymers.

15. A cleansing block composition as claimed in any one of the preceding claims, which is formed by casting.

16. A cleansing block composition as claimed in any one of the preceding claims, wherein the polyethylene glycol distearate is present in an amount of 8 to 35% by weight, guar gum is present in an amount of up to 35% by weight and sodium chloride is present in an amount of up to 64% by weight and the composition further comprises at least one of:

(a) up to 20% by weight of a solid emollient binding agent;
(b) up to 40% by weight of a non-ionic surfactant;
(c) up to 10% by weight of an ethoxylated nonylphenol;
(d) an additional water-soluble salt filler up to a total filler content (including sodium chloride of 64% by weight):
(e) up to 15% by weight of a dye
(f) up to 15% by weight of a cationic quaternary ammonium salt;
(g) up to 15% by weight of a perfume; and
(h) up to 20% by weight of a plasticizer.

17. A cleansing block composition as claimed in Claim 16, comprising:

(a) from 12 to 29% by weight of said polyethylene glycol distearate;
(b) 5 to 15% by weight of guar gum;
(c) 5 to 12% by weight of a solid emollient binding agent;
(d) 20 to 30% by weight of a non-ionic surfactant;
(e) optionally, up to 10% by weight of an ethoxylated nonylphenol;
(f) 20 to 35% by weight of sodium chloride;
(g) 2.5 to 10% by weight of a dye;
(h) optionally, up to 5% by weight of a cationic quaternary ammonium salt;
(i) optionally, up to 10% by weight of a perfume; and
(j) 4 to 15% by weight of a plasticizer.

18. A cleansing block composition as claimed in Claim 16, wherein sodium chloride is present in an amount of up to 32% by weight the composition further comprising:

(i) up to 10% by weight of glyceryl monostearate;

(ii) up to 30% by weight of ethoxylated aliphatic (C$_8$-C$_{20}$) alcohol;
(iii) up to 10% by weight of ethoxylated nonylphenol;
(iv) from 4% to 15% by weight of plasticizer;
(v) up to 32% by weight calcium sulfate; and
(vi) up to 15% by weight of disinfecting agent: coloring agent/or fragrance.

## Patentansprüche

1. Feste Toilettenreinigungsblockzusammensetzung, enthaltend Polyethylenglykoldistearat mit einem Molekulargewicht von 3 000 bis 12 000, ein Bindemittel und ein Füllmittel, dadurch gekennzeichnet, daß das Distearat eine Tropfauflösungszeit (Zeit, die ein vollständig verfestigter Tropfen, der aus der Schmelzphase aus einer 7,5-ml-Pipette verteilt wurde, für die vollständige Auflösung in 75 ml entionisiertem Wasser bei Raumtemperatur benötigt) von mindestens 5,5 Stunden aufweist, das Bindemittel Guargummi ist und das Füllmittel Natriumchlorid ist.

2. Reinigungsblockzusammensetzung nach Anspruch 1, in der das Polyethylenglykoldistearat in einer Menge von 8 bis 35 Gew.-% der Zusammensetzung vorhanden ist.

3. Reinigungsblockzusammensetzung nach Anspruch 2, in der das Poliethylenglykoldistearat in einer Menge von 12 bis 29 Gew.-% vorhanden ist.

4. Reinigungsblockzusammensetzung nach einem der Ansprüche 1 bis 3, in der der Guargummi in einer Menge von 3 bis 35 Gew.-% der Zusammensetzung vorhanden ist.

5. Reinigungsblockzusammensetzung nach Anspruch 4, in der der Guargummi in einer Menge von 5 bis 15 Gew.-% vorhanden ist.

6. Reinigungsblockzusammensetzung nach einem der vorhergehenden Ansprüche, in der Natriumchlorid in einer Menge von 20 bis 64 Gew.-% der Zusammensetzung vorhanden ist.

7. Reinigungsblockzusammensetzung nach Anspruch 6, in der Natriumchlorid in einer Menge von 20 bis 35 Gew.-% vorhanden ist.

8. Reinigungsblockzusammensetzung nach einem der vorhergehenden Ansprüche, in der das Polyethylenglykoldistearat eine Mischung von einem größeren Teil von Polyethylenglykoldistearat mit einem Molekulargewicht zwischen 7 000 und 12 000 und von einem kleineren Teil von Polyethylenglykoldistearat mit einem Molekulargewicht zwischen 3 000 und 7 000 enthält.

9. Reinigungsblockzusammensetzung nach einem der vorhergehenden Ansprüche, weiterhin enthaltend ein festes Erweichungsmittel in einer Menge von bis zu 20 Gew.-% der Zusammensetzung.

10. Reinigungsblockzusammensetzung nach Anspruch 9, in der das feste Erweichungsmittel aus Glycerylmonostearat, Glycerylmonopalmitat, Ethylenglykolstearat und Propylenglykolmonostearat ausgewählt ist.

11. Reinigungsblockzusammensetzung nach einem der vorhergehenden Ansprüche, enthaltend ein oberflächenaktives Mittel, ausgewählt aus ethoxylierten aliphatischen (C$_8$-C$_{20}$)-Alkoholen und ethoxylierten C$_6$-C$_{12}$-Alkylphenolen.

12. Reinigungsblockzusammensetzung nach einem der vorhergehenden Ansprüche, enthaltend eine Mischung aus ethoxylierten Nonylphenolen mit mehr als 20 Mol Ethylenoxid pro Mol Phenol.

13. Reinigungsblockzusammensetzung nach einem der vorhergehenden Ansprüche, enthaltend ein kationisches quaternäres Ammoniumsalz.

14. Reinigungsblockzusammensetzung nach einem der vorhergehenden Ansprüche, enthaltend ein Plastifizierungsmittel, ausgewählt aus Polypropylenglykol, Dipenten, Pinienölfraktionen, d-Limonen und Ethylenoxid/Propylenoxid-Copolymeren.

15. Reinigungsblockzusammensetzung nach einem der vorhergehenden Ansprüche, hergestellt durch Gießen.

16. Reinigungsblockzusammensetzung nach einem der vorhergehenden Ansprüche, in der das Polyethylenglykoldistearat in einer Menge von 8 bis 35 Gew.-% vorhanden ist, Guargummi in einer Menge von bis zu 35 Gew.-% vorhanden ist und Natriumchlorid in einer Menge von bis zu 64 Gew.-% vorhanden ist und die Zusammensetzung weiterhin mindestens einen Bestandteil aus den folgenden enthält:

(a) bis zu 20 Gew.-% eines festen Erweichungs-Bindemittels;
(b) bis zu 40 Gew.-% eines nicht-ionischen oberflächenaktiven Mittels;
(c) bis zu 10 Gew.-% eines ethoxylierten Nonylphenols;
(d) ein zusätzliches wasserlösliches Salz als Füllmittel bis zum Gesamtfüllmittelgehalt (einschließlich Natriumchlorid von 64 Gew.-%);
(e) bis zu 15 Gew.-% eines Farbstoffs;
(f) bis zu 15 Gew.-% eines kationischen quaternären Ammoniumsalzes;
(g) bis zu 15 Gew.-% eines Parfüms; und
(h) bis zu 20 Gew.-% eines Plastifizierungsmittels.

17. Reinigungsblockzusammensetzung nach Anspruch 16, enthaltend:

(a) 12 bis 29 Gew.-% Polyethylenglykoldistearat;
(b) 5 bis 15 Gew.-% Guargummi;
(c) 5 bis 12 Gew.-% eines festen Erweichungs-Bindemittels;
(d) 20 bis 30 Gew.-% eines nicht-ionischen, oberflächen aktiven Mittels;
(e) wahlweise bis zu 10 Gew.-% eines ethoxylierten Nonylphenols;
(f) 20 bis 35 Gew.-% Natriumchlorid;
(g) 2,5 bis 10 Gew.-% eines Farbstoffs;
(h) wahlweise bis zu 5 Gew.-% eines kationischen quaternären Ammoniumsalzes;
(i) wahlweise bis zu 10 Gew.-% eines Parfüms; und
(j) 4 bis 15 Gew.-% eines Plastifizierungsmittels.

18. Reinigungsblockzusammensetzung nach Anspruch 16, in der Natriumchlorid in einer Menge von bis zu 32 Gew.-% der Zusammensetzung vorhanden ist, die weiter enthält:

(i) bis zu 10 Gew.-% Glycerylmonostearat;
(ii) bis zu 30 Gew.-% ethoxylierten aliphatischen $C_8$-$C_{20}$-Alkohol;
(iii) bis zu 10 Gew.-% ethoxyliertes Nonylphenol;
(iv) 4 bis 15 Gew.-% eines Plastifizierungsmittels;
(v) bis zu 32 Gew.-% Calciumsulfat; und
(vi) bis zu 15 Gew.-% eines Desinfektionsmittels, Farbstoffs und/oder Duftstoffs.

**Revendications**

1. Composition de bloc de nettoyage de toilettes sous forme de gâteau solide, comprenant du distéarate de plyéthylèneglycol ayant un poids moléculaire de 3000 à 12 000, un liant et une charge, caractérisée en ce que le distéarate a un temps de dissolution en goutte (temps pour qu'une goutte de phase fondue totalement solidifiée délivrée par une pipette de 7,5 ml se dissolve complètement dans 75 ml d'eau désionisée à la température ambiante) d'au moins 5,5 heures, le liant est la gomme de guar et la charge est le chlorure de sodium.

2. Composition de bloc de nettoyage selon la revendication 1, dans laquelle ledit distéarate de polyéthylèneglycol est présent à raison de 8 à 35% du poids de la composition.

3. Composition de bloc de nettoyage selon la revendication 2, dans laquelle ledit distéarate de polyéthylèneglycol est présent à raison de 12 à 29% en poids.

4. Composition de bloc de nettoyage selon l'une quelconque des revendications 1 à 3, dans laquelle la gomme de guar est présente à raison de 3 à 35% du poids de la composition.

5. Composition de bloc de nettoyage selon la revendication 4, dans laquelle la gomme de guar est présente à raison de 5 à 15% en poids.

6. Composition de bloc de nettoyage selon l'une quelconque des revendications précédentes, dans laquelle le chlorure de sodium est présent à raison de 20 à 64% du poids de la composition.

7. Composition de bloc de nettoyage selon la revendication 6, dans laquelle le chlorure de sodium est présent à raison de 20 à 35% en poids.

8. Composition de bloc de nettoyage selon l'une quelconque des revendications précédentes, dans laquelle ledit distéarate de polyéthylèneglycol consiste en un mélange d'une proportion prépondérante de distéarate de polyéthylèneglycol ayant un poids moléculaire compris entre 7000 et 12 000 et d'une proportion mineure de distéarate de polyéthylèneglycol ayant un poids moléculaire compris entre 3000 et 7000.

9. Composition de bloc de nettoyage selon l'une quelconque des revendications précédentes, comprenant en outre un émollient solide en quantité allant jusqu'à 20% du poids de la composition.

10. Composition de bloc de nettoyage selon la revendication 9, dans laquelle l'émollient solide est choisi entre le monostéarate de glycéryle, le monopalmitate de glycéryle, le stéarate d'éthylèneglycol et le monostéarate de propylèneglycol.

11. Composition de bloc de nettoyage selon l'une quelconque des revendications 1 à 10, comprenant un surfactif choisi parmi des alcools aliphatiques ($C_8$–$C_{20}$) éthoxylés et des (alkyl en $C_6$–$C_{12}$)phénols éthoxylés.

12. Composition de bloc de nettoyage selon l'une quelconque des revendications précédentes, comprenant un mélange de nonylphénols éthoxylés ayant plus de 20 moles d'oxyde d'éthylène par mole de phénol.

13. Composition de bloc de nettoyage selon l'une quelconque des revendications précédentes, comprenant un sel d'ammonium quaternaire cationique.

14. Composition de bloc de nettoyage selon l'une quelconque des revendications précédentes, comprenant un plastifiant choisi entre le polypropylèneglycol, le dipentène, des fractions d'huile de pin, le d-limonène et des copolymères oxyde d'éthylène/oxyde de propylène.

15. Composition de bloc de nettoyage selon l'une quelconque des revendications précédentes, qui est façonnée par coulée.

16. Composition de bloc de nettoyage selon l'une quelconque des revendications précédentes, dans laquelle le distéarate de polyéthylèneglycol est présent à raison de 8 à 35% en poids, la gomme de guar est présente en quantité allant jusqu'à 35% en poids et le chlorure de sodium est présent en quantité allant jusqu'à 64% en poids, et la composition comprend en outre au moins l'un des produits suivants:

(a) jusqu'à 20% en poids d'un agent liant émollient solide;

(b) jusqu'à 40% en poids d'un surfactif non ionique;

(c) jusqu'à 10% en poids d'un nonylphénol éthoxylé;

(d) une charge supplémentaire qui est un sel soluble dans l'eau jusqu'à une teneur totale en charge (y compris le chlorure de sodium) de 64% en poids;

(e) jusqu'à 15% en poids d'un colorant;

(f) jusqu'à 15% en poids d'un sel d'ammonium quaternaire cationique;

(g) jusqu'à 15% en poids d'un parfum; et

(h) jusqu'à 20% en poids d'un plastifiant.

17. Composition de bloc de nettoyage selon la revendication 16, comprenant:

(a) de 12 à 29% en poids dudit distéarate de polyéthylèneglycol;

(b) 5 à 15% en poids de gomme de guar;

(c) 5 à 12% en poids d'un agent liant émollient solide;

(d) 20 à 30% en poids d'un surfactif non ionique;

(e) éventuellement, jusqu'à 10% en poids d'un nonylphénol éthoxylé;

(f) de 20 à 35% en poids de chlorure de sodium;

(g) de 2,5 à 10% en poids d'un colorant;

(h) éventuellement jusqu'à 5% en poids d'un sel d'ammonium quaternaire cationique;

(i) éventuellement jusqu'à 10% en poids d'un parfum; et

(j) 4 à 15% en poids d'un plastifiant.

18. Composition de bloc de nettoyage selon la revendication 7, dans laquelle le chlorure de sodium est présent en quantité allant jusqu'à 32% en poids, la composition comprenant en outre:

(i) jusqu'à 10% en poids de monostéarate de glycéryle;

(ii) jusqu'à 30% en poids d'un alcool aliphatique ($C_8$–$C_{20}$) éthoxylé;

(iii) jusqu'à 10% en poids de nonylphénol éthoxylé;

(iv) de 4% à 15% en poids de plastifiant;

(v) jusqu'à 32% en poids de sulfate de calcium; et

(vi) jusqu'à 15% en poids d'un agent désinfectant, d'un agent colorant et/ou d'un parfum.